(19) Europäisches Patentamt
European Patent Office
Office européen des brevets

(11) Numéro de publication: **0 412 012 A1**

(12) **DEMANDE DE BREVET EUROPEEN**

(21) Numéro de dépôt: **90402206.8**

(51) Int. Cl.$^5$: **A61K 49/02**

(22) Date de dépôt: **01.08.90**

Le titre de l'invention a été modifié (Directives relatives à l'examen pratiqué à l'OEB, A-III, 7.3)

(30) Priorité: **02.08.89 FR 8910411**

(43) Date de publication de la demande:
**06.02.91 Bulletin 91/06**

(84) Etats contractants désignés:
**AT BE CH DE DK ES FR GB GR IT LI LU NL SE**

(71) Demandeur: **MEDGENIX GROUP, S.A.**
**1, place d'Italie**
**B-4020 Liège(BE)**

(72) Inventeur: **Thornback, John R., Dr.**
**Avenue de L'Aulne, 37**
**B-1180 Bruxelles(BE)**
Inventeur: **Bastin, Benoit**
**9a, rue des Hirondelles**
**B-5820 Spy(BE)**
Inventeur: **Joiris, Etienne**
**Avenue E. Cambier 121, Boîte No. 1**
**B-1030 Bruxelless(BE)**

(74) Mandataire: **Warcoin, Jacques et al**
**Cabinet Régimbeau 26, avenue Kléber**
**F-75116 Paris(FR)**

(54) **Marquage de polypeptides au technetium 99m et applications en imagerie.**

(57) La présente invention concerne un procédé de marquage de protéines ou polypeptides au Technétium-99m, caractérisé en ce que

a) on introduit sur ladite protéine des groupes thiols au moyen d'un réactif permettant l'introduction directe de groupes thiols sans étape de réduction de ponts disulfures de la protéine ou polypeptide, et

b) on met en présence la protéine ou le polypeptide ainsi modifié avec du Technétium-99m.

La présente invention a également pour objet les conjugués obtenus par le procédé selon l'invention et l'utilisation de ces conjugués à titre d'agent d'imagerie médicale, ainsi qu'un kit de reconstitution desdits conjugués.

EP 0 412 012 A1

La présente invention concerne un procédé de marquage de protéines ou polypeptides au Technétium-99m, ainsi que des conjugués provenant de la fixation de Technétium-99m sur une protéine ou un polypeptide selon ce procédé et l'utilisation de ces conjugués à titre d'agent d'imagerie médicale. Enfin, la présente invention concerne également un kit de reconstitution desdits conjugués.

La technologie mise au point en 1975 par Kohler et Miestein a permis la production de nombreux anticorps monoclonaux. L'usage de ces derniers a révolutionné les méthodes de diagnostic utilisées dans le domaine médical, que ce soit au niveau du diagnostic in vitro ou au niveau de l'imagerie médicale.

Pour cette dernière, il s'est avéré nécesaire de mettre au point de nouvelles méthodes permettant la fixation sur les anticorps des isotopes couramment utilisés en imagerie. Diverses méthodologies ont ainsi été développées afin de permettre le marquage des protéines à l'iode-131, à l'iode-123, à l'indium-111, au Technétium-99m et, dans une moindre mesure, au gallium-67. Parmi ces isotopes, le Technétium-99m occupe une place de choix pour diverses raisons. D'une part, il présente des caractéristiques physiques idéales pour l'utilisation en imagerie médicale : l'énergie de son rayonnement

$$\gamma (140\ keV)$$

permet une excellente résolution des images, et sa courte demi-vie (T = 6,02 h) assure une irradiation minimum des patients. D'autre part, l'existence de générateurs a rendu cet isotope très disponible et très peu coûteux. C'est ce qui explique l'intérêt considérable accordé aux méthodes de marquage des protéines au Technétium-99m.

La méthode la plus simple consiste à réduire le pertechnétate radioactif ($TcO_4^-$) élué du générateur par addition d'un réducteur, en général un sel d'étain II, en présence de la protéine à marquer, sur laquelle le technétium réduit peut alors se fixer. La force avec laquelle le technétium est fixé aux sites de liaisons naturels présents sur la protéine reste cependant très faible, posant de gros problèmes de stabilité du marquage in vitro et in vivo. C'est pourquoi, il s'est avéré nécessaire d'une part, d'introduire sur la protéine des structures ou des groupes fonctionnels présentant une affinité élevée sur le Technétium, sans altérer les propriétés biologiques de la protéine, et d'autre part, d'éviter la fixation du Technétium sur les sites naturels de faible affinité présents sur la protéine tels que OH ou $NH_2$.

Deux voies d'approches permettent de générer sur la protéine des sites de haute affinité pour le Technétium. La première de ces voies consiste à fixer sur la protéine des structures permettant la chélation multivalente du Technétium. A côté du DTPA (D.J. Hnatowich, W.W. Layne and R.J. Childs Int. J. Appl. Radiat. Isot., 1981, <u>33</u> , 327-332) dont l'affinité pour le Technétium s'est révélée insuffisante, diverses structures ont été essayées, en particulier les ligands de type $N_2S_2$ (A.R. Fritzberg, Nucl. Med. (Stuttgart) 1987, <u>26</u> , 7-12). Ces chélates peuvent être fixés sur la protéine avant ou après la chélation du Technétium.

La seconde voie d'approche consiste à faire apparaître sur la protéine des groupes thiols libres. Ceux-ci ont en effet une affinité élevée pour le Technétium réduit. Les méthodes couramment utilisées pour faire apparaître ces groupes thiols consistent en une réduction des ponts disulfures initialement présents sur la protéine en groupes thiols libres (A. Schwartz and A. Steinstrasser, J. Nucl. Med., 1987, <u>26</u> , 7-12). Divers réducteurs ont été utilisés à cette fin, parmi lesquels l'Etain II, en concentration importante avec des incubations prolongées, le β-mercaptoethanol, la mercaptoéthylamine et le Dithiothréitol. L'inconvénient majeur de cette approche provient du fait que la rupture des ponts disulfure altère la structure tertiaire et/ou quaternaire de la protéine, modifiant ainsi ses propriétés biologiques. En particulier, lors du traitement réducteur des fragments $F(ab')_2$ des anticorps, il se produit une réduction partielle en F = ab, ce dernier se marquant alors préférentiellement, ce qui est d'autant plus gênant que l'activité immunologique des $F(ab')_2$ est en général de loin inférieure à celle des $F(ab')_2$.

Des réactifs tels que le SPDP ont été proposés (I.F.C. McKenzie, G.A. Pietersz and J. Kanellos, WO. Patent 87/04164) pour l'introduction de ponts disulfures nouveaux susceptibles de générer des groupes thiols après une étape de réduction desdits ponts disulfures. Ce type de méthode présente donc le même inconvénient majeur, c'est-à-dire un risque d'altération de la structure de la protéine.

Le but de la présente invention est donc de proposer un procédé de marquage de protéines ou polypeptides avec du Technétium-99m qui soit suffisamment stable et qui n'altère pas la structure et les propriétés biologiques de la protéine ou du polypeptide.

Pour ce faire, la présente invention a pour objet un procédé de marquage de protéines ou polypeptides au Technétium-99m, caractérisé en ce que

a) on introduit sur ladite protéine des groupes thiols au moyen d'un réactif permettant d'introduction directe de groupes thiols sans étape de réduction de ponts disulfures de la protéine ou polypeptide, et

b) on met en présence la protéine ou le polypeptide ainsi modifié avec du Technétium-99m.

Dans un mode particulier de réalisation du procédé selon l'invention, on introduit lesdits groupes thiols

par réactions dudit réactif sur les fonctions amine primaires de la protéine, en particulier la fonction amine $\epsilon$ des lysines de la protéine ou du polypeptide.

On peut citer comme réactif permettant l'introduction sur ladite protéine ou ledit polypeptide de groupes thiol, des réactifs soufrés possédant un groupement activé, en particulier les 2'iminothiolane.

Le 2-iminothiolane par exemple disponible sous forme de sel chlorure réagit principalement avec les fonctions amines primaires des protéines selon le schéma réactionnel suivant :

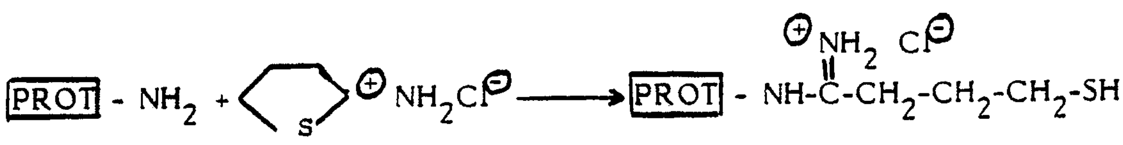

A côté de l'introduction de groupes fonctionnels de haute affinité sur la protéine, il demeure important d'éviter la fixation non spécifique de Technétium sur les sites de faible affinité naturellement présents sur la protéine. A cette fin, selon l'invention, on présente le Technétium à la protéine sous la forme d'un complexe technétié d'affinité intermédiaire, échangeant le Technétium avec les sites de haute affinité introduits sur la protéine, mais pas avec les sites de basse affinité. En outre, de tels complexes ne doivent pas avoir l'inconvénient d'être éliminés lentement de l'organisme. La fraction du Technétium ne s'échangeant pas avec la protéine pourrait alors interférer avec l'image obtenue pour la protéine technétiée. C'est pourquoi, selon la présente invention, on utilise avantageusement comme complexe intermédiaire du glucoheptonate technétié, dont d'éventuelles traces sont rapidement éliminées par excrétion urinaire.

On prépare le complexe de Technetium d'affinité intermédiaire tel que le complexe de Technetium avec du glucoheptonate, que l'on ajoute à la protéine ou au polypeptide modifié par introduction de groupes thiols au moyen d'un réactif tel que le 2-iminothiolane.

Dans le procédé selon l'invention, classiquement le Technétium-99m peut provenir de la réduction notamment par un sel d'Etain tel que du chlorure de pertechnétate $TcO_4^-$.

Le procédé selon la présente invention est particulièrement intéressant lorsqu'il s'agit de marquer un anticorps ou un fragment $F(ab')_2$ d'anticorps.

La présente invention a également pour objet des conjugués provenant de la fixation de Technétium-99m sur une protéine ou un polypeptide sur lesquels on a introduit des groupes thiols selon le procédé de la présente invention. Ce type de conjugué est particulièrement utile à titre d'agent d'imagerie médicale.

Enfin, la présente invention a également pour objet un kit de reconstitution de conjugué selon l'invention, caractérisé en ce qu'il comprend

a) une protéine ou un polypeptide sur lesquels des groupes thiols ont été introduits au moyen d'un réactif permettant l'introduction directe de groupes thiols sans étape de réduction de ponts disulfures,

b) un agent réducteur de pertechnétate, et éventuellement,

c) un composé susceptible de complexer le Technétium-99m ayant une affinité intermédiaire tel qu'il échange le Technétium avec les sites SH de haute affinité introduit sur la protéine mais pas avec les sites naturelles de faible affinité, et enfin

d) du pertechnétate de Technétium radioactif.

D'autres caractéristiques et avantages de la présente invention apparaîtront à la lumière de la description détaillée d'un mode de réalisation de celle-ci qui va suivre.

La méthode de marquage de protéines au Technétium-99m détaillée ci-après consiste en l'introduction de groupes thiols libres sur lesdites protéines par réaction des lysines de ces protéines avec le 2-iminothiolane. Le Technétium est ensuite échangé par du glucoheptonate technétié et ces groupes thiols.

La description qui va suivre est faite en référence à des dessins sur lesquels

- La figure 1 représente l'analyse HPLC de la BSA modifiée par le réactif 2-iminothiolane.
- La figure 2 représente l'analyse HPLC de l'IgGI 15C5 modifié par le réactif 2-iminothiolane.
- La figure 3 représente l'analyse HPLC du $F(ab')_2$ 15C5, IgGI modifié par le 2-iminothiolane.
- La figure 4 représente l'analyse HPLC du $F(ab')_2$ 15C5 marqué au Technétium. — : profil UV à 260 nm o-------o : radioactivité

## 1. Réaction des protéines avec le 2-iminothiolane (= méthyl 4 mercaptobutirimidate)

Le 2-iminothiolane réagit avec les fonctions amines primaires des protéines selon le schéma réactionnel suivant.

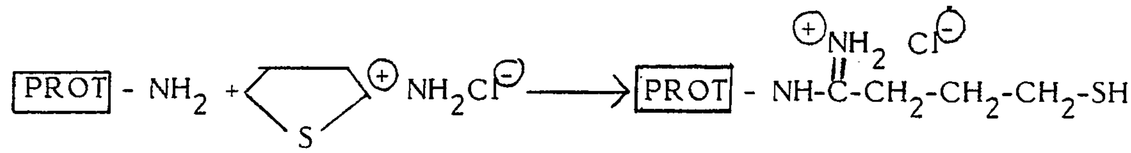

Ce type de réaction a été utilisé pour l'albumine bovine, un anticorps monclonal de souris (15C5, IgGl) et le fragment F(ab′)2 de cet anticorps.

1.2 albumine bovine

Une solution de 2-iminothiolane est préparée extemporanément dans un tampon0,1 M phosphate, pH 7,4. La concentration en iminothiolane est de 40 mM. A 5 mg d'albumine bovine (BSA grade V, Sigma) en solution dans 1 ml de tampon 0,1 M phosphate, pH 7,4, on ajoute goutte à goutte 0,25 ml de solution de 2-iminothiolane en maintenant une agitation légère. On réalise ainsi un rapport molaire iminothiolane/BSA de 333/1. La réaction se poursuit pendant 12 h à température ambiante à l'abri de l'oxygène. La protéine modifiée est ensuite séparée de l'excès d'iminothiolane par passage sur une colonne Séphadex G50 (20 x 1,5 cm), équilibrée dans le tampon phosphate 0,1 M, pH 7,4. Ce même tampon est utilisé pour l'élution.

Le profil obtenu après séparation (analyse HPLC : filtration molélculaire sur 2 colonnes placées en série : Du Pont Zorbax et FG 250) est montré à la figure 1.

b) Anticorps monoclonal de souris

L'anticorps 15C5 (IgGl) reconnaît le fragment D-Dimère de la fibrine humaine. Il est préparé à partir de liquide d'ascite par purification Sépharose - Protéine A.

Une solution de 2-iminothiolane 40 mM est préparée extemporanément dans un tampon 0,1 M phosphate, pH 7,4. A 5 mg d'anticorps en solution dans 1 ml de tampon phosphate, on ajoute goutte à goutte 0,5 ml de solution de 2-iminothiolane en maintenant une agitation légère, réalisant un rapport molaire iminothiolane/anticorps de 600/1.

La réaction se poursuit pendant 12 h à température ambiante, à l'abri de l'oxygène. La protéine modifiée est ensuite séparée de l'excès d'iminothiolane par passage sur une colonne séphadex G50 (20 x 1,5 cm) équilibrée dans un tampon 0,1 M citrate, pH 5,6. Ce même tampon est utilis pour l'élution.

L'anticorps est ensuite réparti dans des fioles par fractions de 0,5 mg lyophilisé. La redissolution par addition d'eau au produit lyophilisé est rapide et complète. Le profil obtenu après dissolution est présenté à la figure 2 (analyse HPLC : filtration moléculaire sur 2 colonnes placées en série : Du Pont Zorbox et GF 250).

Le produit est lyophilisé et conservé à 4° C.

c) Fragments F(ab′)2 d'un anticorps monoclonal de souris

Le fragment F(ab′)2 de l'anticorps 15C5 est préparé par digestion de cet anticorps par la papaïne, en absence de cystéine, puis purifié par filtration moléculaire (AcA 4/4) et échange d'ion (DEAE).

Une solution de 2-iminothiolane 40 mM est préparée extemporanément dans un tempon 0,1 M phosphate, pH 7,4. A 5 mg de fragment F(ab′)2 en solution dans 1 ml de tampon phosphate, on ajoute goutte à goutte 0,25 ml de solution d'iminothiolane en maintenant une agitation légère, réalisant un rapport molaire iminothiolane/F(ab′)2 de 200/1. La réaction se poursuit pendant 3 h à 4°, à l'abri de l'oxygène. La protéine modifiée est ensuite traitée de façon similaire à l'anticorps entier purification et lyophilisation. Le profil obtenu après dissolution du produit lyophilisé est présenté à la figure 3 (analyse HPLC : filtration moléculaire sur une colonne TSK 3000 SW).

Le produit lyophilisé est conservé à 4°.

2. Préparation du réactif de transchélation

100 ml de solution de glucoheptonate de sodium à 25 mg/ml et 100 ml de solution de $SnCl_2$ à 0,05

mg/ml sont préparés extemporanément dans de l'eau dégazée et mélangés. La solution obtenue est répartie par fractions de 0,4 ml dans des fioles et lyophilisée.

Chaque fiole contient donc 5 mg de glucoheptonate de Na et 0,01 mg de $SnCl_2$. Après addition de 1 ml de Nacl 0,9 %, on obtient rapidement une solution limpide à pH 6,5.

3. Marquage au Technétium-99m des protéines modifiées par le 2-iminothiolane

a) Dans un premier temps, on effectue le marquage du réactif de transchélation en ajoutant à une fiole de glucoheptonate lyophilisé 1 ml de solution contenant 10-100 mCi de $^{99m}TcO_4^-$ dans du NaCl 0,9 %. Cette solution de $TcO_4^-$ est obtenue en éluant un générateur de Technétium avec du NaCl 0,9 %, puis en diluant éventuellement cette solution avec du NaCl 0,9 % de façon à obtenir l'activité volumique (nombre de mCi/ml) désirée.

Après 1/2 heure (d'incubation à température ambiante, la radioactivité est présente exclusivement sous forme de glucoheptonate technétié (analyse par CCM : 5 µl de solution sont déposés sur un support ITLC-SG (Gelmann). La migration est effectuée dans du NaCl 0,9 % pour la détection des colloides ($R_F$ = 0) et dans de la Méthyl Ethyl Cétone pour la détection du pertechnétate ($R_F$ = 1).

b) La protéine est resolubilisée en ajoutant 0,5 ml d'eau au flacon de protéine lyophilisée. On obtient ainsi une solution à 1 mg/ml dans un tampon citrate 0,1 M, pH 5,6. Dans le cas de l'albumine bovine, qui n'a pas été lyophilisée, on dilue la solution de protéine par du tampon phosphate pH 7,4 de façon à obtenir une concentration de 1 mg/ml.

c) On ajoute ensuite à la protéine la quantité de radioactivité souhaitée (10-100 mci/mg), sous la forme d'un volume variable de solution de glucoheptonate technétié. Le mélange est laissé à température ambiante à l'abri de l'oxygène pendant 1 h 30.

d) Différentes analyses sont ensuite effectuées :

- détection des colloides : par mesure de l'activité volumique avant et après filtration de la solution sur un filtre 0,22 µm (Millex).
- détection du pertechnétate par CCM : 5 µl de solution sont déposés sur un support ITLC-SG. La migration est effectuée dans de la Méthyl Ethyl Cétone. Dans ces conditions, seul le pertechnétate migre au front du solvant, tandis que les colloides, la protéine et le glucoheptonate technétié restent au point de dépôt.
- détection du glucoheptonate technétié résiduel par CCM : 5 µl de solution sont déposés sur un support ITLC-SG. La migration est effectuée dans du Na Cl 0,9 %. Dans ces conditions, le pertechnétate et le glucoheptonate technétié migrent au front de solvant tandis que les colloides et la protéine restent au point de dépôt. Le pourcentage de glucoheptonate résiduel est obtenu par soustraction du résultat obtenu pour le pertechnétate dans l'analyse précédente.

Les résultats obtenus sont détaillés dans le tableau suivant.

| | colloides | pertechnétate | glucoheptonate technétié | protéine |
|---|---|---|---|---|
| Albumine bovine | <1 % | 3 % | 60 % | 37 % |
| IgG 15C5 | <1 % | 2 % | 1 % | 97 % |
| F(ab')$_2$ 15C5 | <1 % | 10 % | 15 % | 75 % |

En résumé, on obtient un marquage de la protéine variant de 40 à 95 % de la radioactivité en jeu, le reste de l'activité étant présent sous forme de pertechnétate ou de glucoheptonate technétié.

e) La radioactivité non liée à la protéine peut ensuite être séparée de la protéine par filtration moléculaire sur Séphadex 650.

L'analyse en HPLC (filtration moléculaire sur colonne TSK 3000 SW) effectuée après cette purification permet de montrer que le pic de radioactivité est associé au pic de la protéine. A la figure 4, on présente les résultats obtenus pour ce type d'analyse dans le cas du fragment F(ab')$_2$ 15C5 marqué au Technétium.

4. Analyse de la stabilité des marquages

a) Stabilité in vitro

Des analyses en ITLC similaires à celles décrites ci-dessus ont été effectuées à intervalles réguliers sur les produits marqués pendant une période de 24 h suivant le marquage, les produits étant conservés à température ambiante et en présence d'oxygène. Dans aucun cas, un relarguage de pertechnétate ou l'apparition colloides n'ont pu être détectés dans des proportions supérieures à 5 % du total de la radioactivité. L'addition d'EDTA à la concentration de 0,2 M ne modifie pas ces résultats.

Les analyses HPLC effectuées pendant ce laps de temps ne mettent en évidence aucune modification par rapport aux analyses effecutées directement après marquage.

b) Stabilité in vivo

0,5 mCi d'albumine marquée au technétium-99m par la méthode décrite ci-dessus avec une activité spécifique de 10 mCi/mg sont injectés à un rat, dans la veine fémorale. L'animal est sacrifié 1 heure après l'injection et une biodistribution est effectuée. La persistance de l'activité au niveau du sang (>50 % de la dose injectée) et la faible accumulation hépatique (<10 % de la dose injectée) permettent de vérifier qu'il n'y a pas formation d'un colloide technétié après injection.

5. Analyse de l'immunoréactivité de l'anticorps et du fragment $F(ab')_2$ marqués

Des tests de fixation de l'anticorps 15C5 et de son fragment $F(ab')_2$ marqués au Technétium-99m par la méthode décrite ci-dessus ou à l'Iode-123 (J.C. Speck, Biochem, Biophys. Res. Commun., 1978, 80 , 849) par une méthode classique (Iodogène) sont effectués de la façon suivante :

- l'antigène reconnu par l'anticorps 15C5 est le fragment D-Dimère de la fibrine humaine. On applique 50 µl d'une solution de fragment D-Dimère à la concentration de 10 µg/ml dans un tampon 0,01 M phosphate, 0,15 M NaCl, pH 7,2 dans chaque puit d'une microplaque sécable (Costar). - Après incubation de 3 heures à température ambiante, les microplaques sont lavées, puis saturées par une incubation de 1 heure avec 200 yl/puit d'une solution d'albumine bovine à 1 % p/v dans le tampon phosphate.
- Après élimination de la solution d'albumine, on introduit 50 µl d'une solution d'anticorps ou de fragment marqué dont la concentration varie de 37 à 0,3 nM. L'expérience est réalisée en triple.
- Après 1 h d'incubation à température ambiante, les plaques sont lavées 5 fois par un tampon 0,01 M phosphate, 0,15 M NaCl, pH 7,2 contenant 0,005 % de Tween 20. L'activité associée à chaque puit est ensuite déterminée par comptage gamme.
- L'analyse par la méthode de Scatchard des paramètres de fixation ainsi déterminés permet de calculer la constante de dissociation de l'anticorps et de son fragment. Les valeurs obtenues pour les produits technétié sont respectivement de $1{,}75.10^{-9}$M pour l'anticorps entier IgGI et de $2{,}25.10^{9}$M pour le fragment $F(ab')_2$. Ces valeurs sont comparables à celles obtenues pour les produits marqués à l'iode radioactif.

**Revendications**

1. Procédé de marquage de protéines ou polypeptides au Technetium-99m, caractérisé en ce que
   a) introduit sur ladite protéine des groupes thiols au moyen d'un réactif permettant l'introduction directe de groupes thiols sans étape de réduction de ponts disulfures de la protéine ou polypeptide, et
   b) on met en présence la protéine ou le polypeptide ainsi modifié avec du Technétium-99m.
2. Procédé selon la revendication 1, caractérisé en ce qu'on introduit lesdits groupes thiols par réaction dudit réactif sur des fonctions amine primaires $\epsilon$ de la protéine ou du polypeptide.
3. Procédé selon la revendication 1 ou 2, caractérisé en ce qu'on introduit sur ladite protéine ou ledit polypeptide les groupes thiols au moyen d'un réactif soufré possédant un groupement activé.
4. Procédé selon l'une des revendications 1 à 3, caractérisé en ce qu'on introduit lesdits groupes thiols par réaction d'un réactif 2-iminothiolane avec la protéine ou le polypeptide.
5. Procédé selon l'une des revendications précédentes, caractérisé en ce qu'à l'étape b) le Technétium-99m est présenté sous forme de complexe de Technétium-99m ayant une affinité intermédiaire telle qu'il échange le Technétium avec les sites SH de haute affinité introduits sur la protéine selon l'étape a) et non avec les sites de basse affinité naturelle présents sur la protéine ou le polypeptide, et complexe qui s'élimine rapidement de l'organisme.

6. Procédé selon la revendication 5, caractérisé en ce que le complexe de Technétium est un glucoheptonate de Technétium-99m.

7. Procédé selon l'une des revendications précédentes, caractérisé en ce que le Technétium-99m provient de la réduction de pertechnétate notamment par un sel d'étain (II).

8. Procédé selon l'une des revendications précédentes, caractérisé en ce que la protéine consiste en un anticorps ou un fragment $F(ab')_2$ d'anticorps.

9. Conjugué provenant de la fixation de Technetium-99m sur une protéine ou un polypeptide obtenu par le procédé selon l'une des revendications précédentes.

10. Utilisation du conjugué selon la revendication 9 à titre d'agent d'imagerie médicale.

11. Kit de reconstitution de conjugué selon la revendication 9, caractérisé en ce qu'il comprend

a) une protéine ou un polypeptide sur lesquels des groupes thiols ont été introduits au moyen d'un réactif permettant l'introduction directe de groupes thiols sans étape de réduction de ponts disulfures,

b) un agent réducteur de pertechnétate, et éventuellement,

c) un composé susceptible de complexer le Technétium-99m ayant une affinité intermédiaire tel qu'il échange le Technétium avec les sites SH de haute affinité introduit sur la protéine mais pas avec les sites naturelles de faible affinité, et enfin

d) du Pertechnétate de Technétium radioactif.

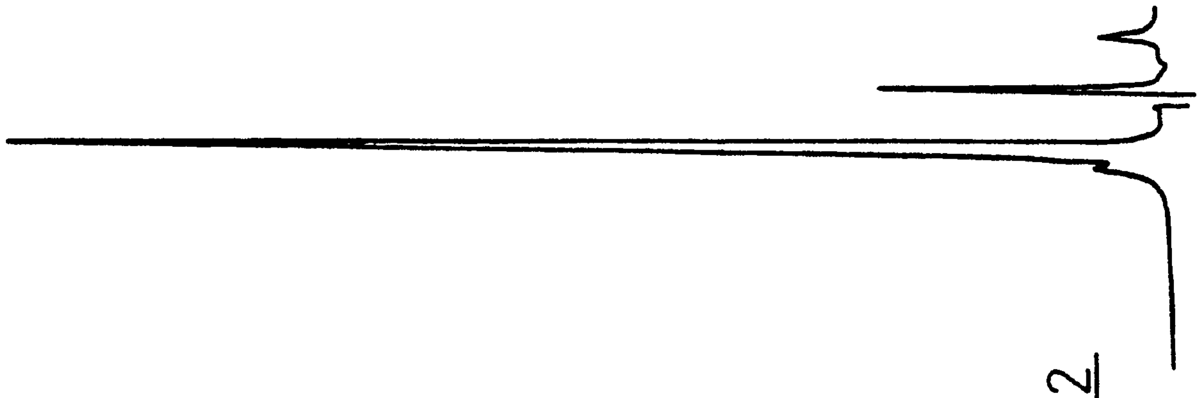

FIG_2

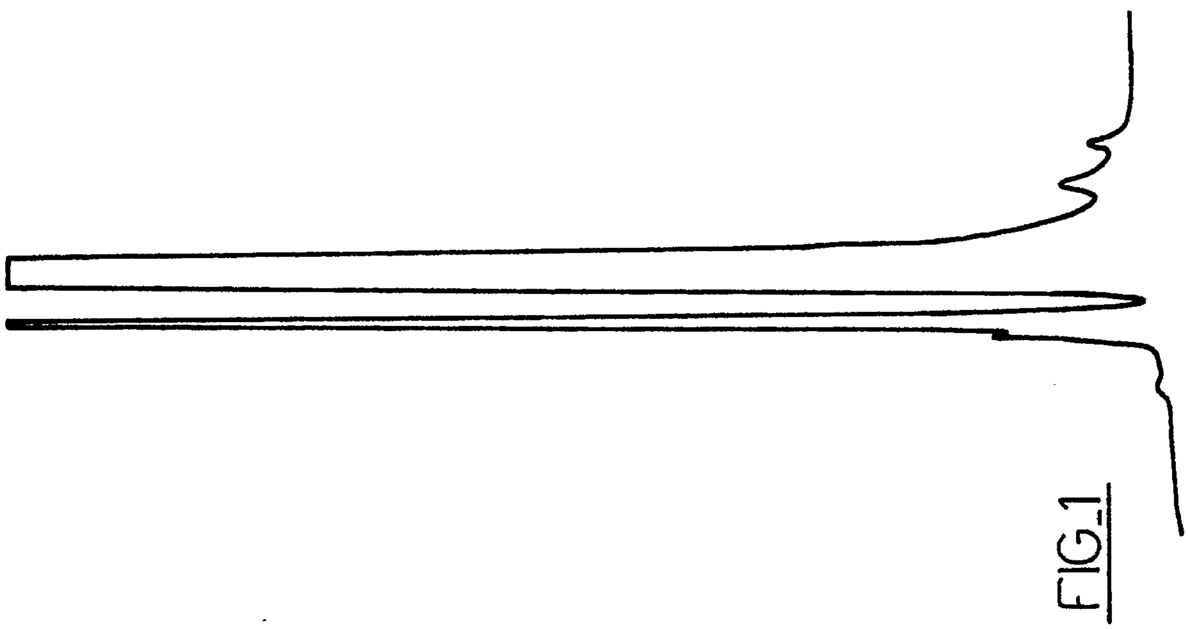

FIG_1

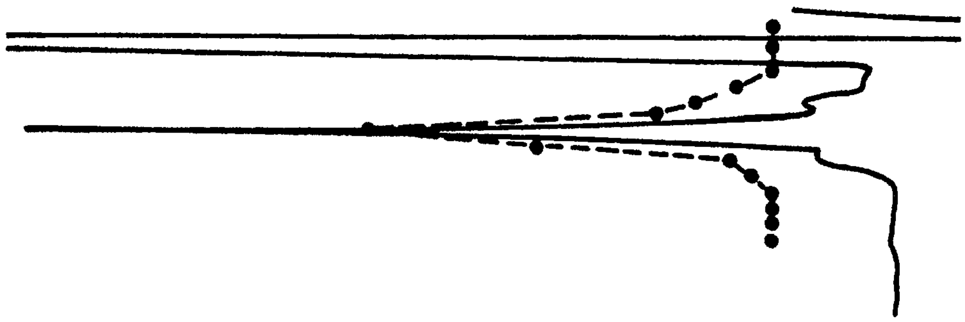

FIG_4

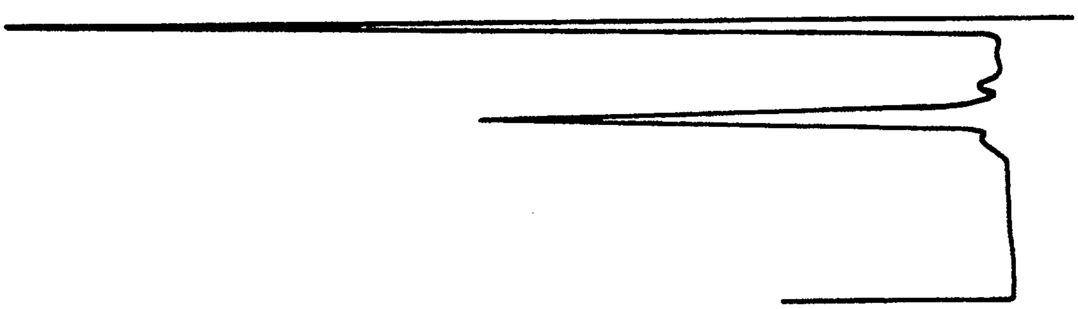

FIG_3

Office européen des brevets

# RAPPORT DE RECHERCHE EUROPEENNE

Numero de la demande

EP 90 40 2206

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | TETRAHEDRON LETTERS, vol. 30, no. 15, 1989, pages 1885-1888, Pergamon Press Plc, GB; H.K. MISRA et al.: "Synthesis of a novel diaminodithiol ligand for labeling proteins and small molecules with technetium-99m"<br>* Page 1885; page 1886, alinéa 2 *<br>--- | 1-3,8-10 | A 61 K 49/02 |
| X | S.T.N. INTERNATIONAL (Karlsruhe), FILE CA & CHEMICAL ABSTRACTS, vol. 109, no. 17, résumé no. 145270s, Columbus, Ohio, US; A.R. FRITZBERG et al.: "Specific and stable labeling of antibodies with technetium-99m with a diamide dithiolate chelating agent"<br>* Résumé entier *<br>--- | 1-5,8-10 | |
| X | S.T.N. INTERNATIONAL (Karlsruhe), FILE BIOSIS & BIOSIS, vol. 85, résumé no. 329012, Columbus, Ohio, US; A. DAVISON et al.: "The preparation characterization and crystal and molecular structure of oxobisethan-1-2-dithiolato-SMU-S'technetium-V-oxoethan-12-dithiolate-S S'technetium-V A technetium-V-compound containing metal-sulfur-metal bridges", & CAN. J. CHEM. 63(2), 1985, 319-323<br>* Résumé entier *<br>--- -/- | 1-5,8-10 | DOMAINES TECHNIQUES RECHERCHES (Int. Cl.5)<br>A 61 K |

Le présent rapport a été établi pour toutes les revendications

| Lieu de la recherche | Date d'achèvement de la recherche | Examinateur |
|---|---|---|
| LA HAYE | 02-10-1990 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 90 40 2206

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | S.T.N. INTERNATIONAL (Karlsruhe) FILE CA & CHEMICAL ABSTRACTS, vol. 108, no. 1, résumé no. 2685z, Columbus, Ohio, US; J. FRANZ et al.: "The production of technetium-99m-labeled conjugated antibodies using a cyclam-based bifunctional chelating agent", & NUCL. MED. BIOL., 14(6), 569-72<br>* Résumé entier *<br>--- | 1-5,8-10 | |
| Y | US-A-4 652 440 (C.H. PAIK)<br>* Colonne 2, lignes 33-53; colonne 3, lignes 62-67; colonne 5, lignes 26-50; colonne 7, lignes 41-66; colonne 8, lignes 39-43 *<br>----- | 1-11 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 02-10-1990 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)